(19) Europäisches Patentamt
European Patent Office
Office européen des brevets

(11) Veröffentlichungsnummer : **0 128 285**
**B1**

(12) **EUROPÄISCHE PATENTSCHRIFT**

(45) Veröffentlichungstag der Patentschrift : **29.07.87**

(51) Int. Cl.⁴ : **C 07 D211/58**

(21) Anmeldenummer : **84103702.1**

(22) Anmeldetag : **04.04.84**

# (54) Verfahren zur Herstellung von Polyalkylpiperidylaminen.

(30) Priorität : **13.06.83 DE 3321332**

(43) Veröffentlichungstag der Anmeldung : **19.12.84 Patentblatt 84/51**

(45) Bekanntmachung des Hinweises auf die Patenterteilung : **29.07.87 Patentblatt 87/31**

(84) Benannte Vertragsstaaten : **AT BE CH DE FR GB IT LI LU NL SE**

(56) Entgegenhaltungen :
**EP-A- 0 033 663**
**EP-A- 0 061 785**
**DE-A- 3 007 996**
**GB-A- 834 290**

(73) Patentinhaber : **HÜLS AKTIENGESELLSCHAFT**
**Patentabteilung / PB 15 - Postfach 13 20**
**D-4370 Marl 1 (DE)**

(72) Erfinder : **Disteldorf, Josef, Dr.**
**Am Sengenhoff 2 a**
**D-4690 Herne 1 (DE)**
Erfinder : **zur Hausen, Manfred, Dr.**
**Höchster Strasse 1**
**D-4370 Marl (DE)**
Erfinder : **Hübel, Werner, Dr.**
**Birnenbruchstrasse 34**
**D-4690 Herne 1 (DE)**
Erfinder : **Kriebel, Günter, Dr.**
**Gaussstrasse 15**
**D-4690 Herne 2 (DE)**

**EP 0 128 285 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Gegenstand dieser Erfindung ist ein Verfahren zur Herstellung von Polyalkylpiperidylaminen der allgemeinen Formel I

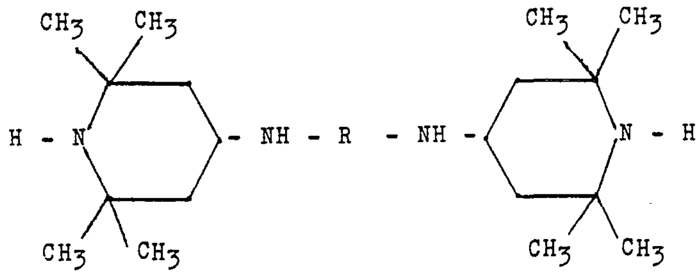

(I)

in der R für einen $C_{2-18}$ Alkylenrest, der $C_{1-3}$-alkylsubstituiert sein kann, steht.

Diese Verbindungen sollen nach der Zahl der Piperidyleinheiten, die sie enthalten, als « dimere » bezeichnet werden und dadurch von den Piperidylaminen, die nur eine Piperidyleinheit enthalten (monomer) oder mehr als vier Piperidyleinheiten enthalten (polymer) unterschieden werden.

Polyalkylpiperidylamine stellen wertvolle Zwischenprodukte, insbesondere für die Synthese von Kunststoffadditiven, dar.

Die Herstellung von Polyalkylpiperidylaminen durch reduktive Aminierung von Polyalkylpiperidonen-4 ist Stand der Technik.

So ist aus der GB-PS 834 290 ein Verfahren bekannt, wonach 2,2,6,6-Tetramethylpiperidon-4 in Gegenwart eines Platin/Aktivkohle-Katalysators und in Anwesenheit von Wasserstoff mit Dialkylaminoalkylaminen zu den in 4-Stellung substituiertem 2,2,6,6-Tetramethylpiperidin umgesetzt wird.

In der DE-OS-2 349 962 wird die reduktive Aminierung von 1-Benzyl-2,2,6,6-tetramethylpiperidon-4 mit Butylamin in Methanol an einem Platinkatalysator beschrieben.

Gegenstand der DE-OS-2 611 208 ist die reduktive Aminierung von 2,2,6,6-Tetraalkylpiperidon-4 mit Alkylendiaminen an Pt-Kontakten und die Umsetzung der erhaltenen dimeren Polyalkylpiperidylamine mit bifunktionellen Verbindungen, die Halogen- und/oder Epoxygruppen enthalten, zu polymeren Polyalkylpiperidylaminen.

In der DE-OS-2 621 870 wird die reduktive Aminierung von 2,2,6,6-Tetramethylpiperidon-4 in Gegenwart eines Raney-Nickel-Katalysators beschrieben.

Schließlich beschreibt die DE-OS-3 007 996 die reduktive Aminierung von 2,2,6,6-Tetramethylpiperidon-4 mit Mono- und Diaminen an Cobalt- und Nickelkatalysatoren. In dieser Schrift wird empfohlen, das bei der Kondensation gebildete Reaktionswasser vor Beginn der Hydrierung durch azeotrope Destillation zu entfernen.

Nachteilig an den drei erstgenannten Verfahren ist die Verwendung teurer Edelmetallkatalysatoren, die nicht verlustfrei regeneriert werden können.

Raney-Katalysatoren, wie sie im vierten Verfahren eingesetzt werden, sind grundsätzlich für den Chargenbetrieb bestimmt. Eine Wiederverwendung ist umständlich, der Einsatz in kontinuierlichen Verfahren erfordert einen erheblichen Aufwand.

Wie das letztgenannte Verfahren aufzeigt, ist es bei der reduktiven Aminierung von 2,2,6,6-Tetramethylpiperidon-4 von Vorteil, wenn das Reaktionswasser möglichst rasch aus dem Reaktionsgemisch entfernt wird. Der Grund hierfür liegt in der leichten Hydrolysierbarkeit der 2,2,6,6-Tetramethylpiperidon-4, insbesondere bei höheren Temperaturen, die naturgemäß zu Ausbeuteverlusten führen muß.

Eine grundsätzlich andere Möglichkeit der Herstellung von Polyalkylpiperidylaminen besteht in der Umsetzung von 4-Amino-polyalkylpiperidinen mit Dihalogenverbindungen in Gegenwart von Alkaliverbindungen zur Neutralisation des freiwerdenden Halogenwasserstoffs (vgl. DE-OS-2 611 208). Wenn auch bei dieser Arbeitsweise hohe Ausbeuten an Polyalkylpiperidylaminen erzielt werden, so stellt doch der Einsatz von Alkaliverbindungen und der Anfall von erheblichen Mengen an Salzen einen erheblichen Nachteil dar. Aufgrund der hohen Wasserlöslichkeit der Polyalkylpiperidylamine treten erhebliche Verluste der Reaktionsprodukte bei der Abtrennung aus dem Reaktionsgemisch auf.

Eigene Versuche, die dimeren Polyalkylpiperidylamine durch hydrierende Alkylierung von Diaminen mit in 4-Stellung hydroxylierten Polyalkylpiperidinen herzustellen, lieferten völlig unbefridigende Ergebnisse. Unter den Reaktionsbedingungen kommt es nämlich zu Reaktionen der Diamine untereinander und miteinander. Beispielsweise erhielt man beim Einsatz von Ethylendiamin Piperazin und beim Einsatz von Hexamethylendiamin Azacycloheptan.

Daher erschien es aussichtslos, mit dem Verfahren der hydrierenden Alkylierung von Aminen (vgl. Houben-Weyl, Band XI/1, Seite 126 ff.) einen Durchbruch zu erzielen.

Ziel der Erfindung war es, ein neues Verfahren zur Herstellung dimerer Polyalkylpiperidylamine zu entwickeln, wobei man von 4-Amino-2,2,6,6-tetramethyl piperidin der Formel II

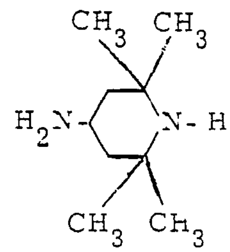

ausgeht.

Dabei wurde überraschend gefunden, daß man durch reduktive Alkylierung von 4-Amino-2,2,6,6-tetramethylpiperidin mit difunktionellen Alkoholen die gewünschten dimeren Polyalkylpiperidylamine erhält.

Das erfindungsgemäße Verfahren verläuft nach folgendem Reaktionsschema :

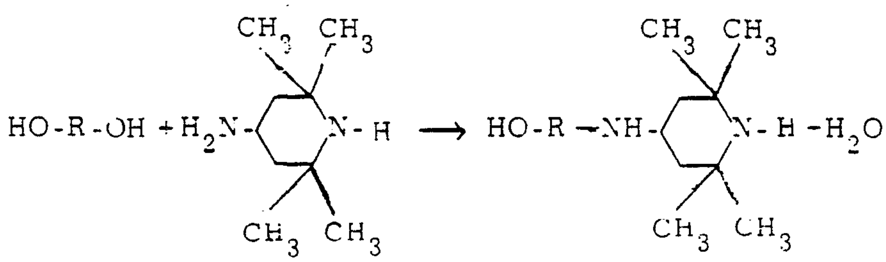

(II) (III)

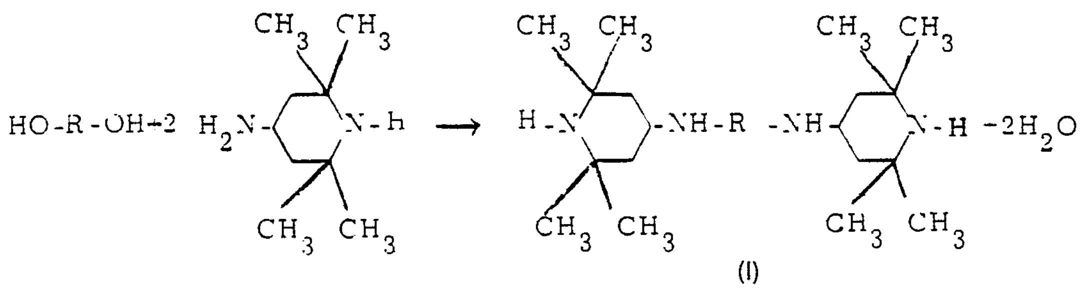

(I)

Je nachdem, in welchem Molverhältnis die Umsetzung erfolgt, erhält man unterschiedliche Gemische der Verbindungen I und III. Durch Rückführung von III kann die Reaktion so gelenkt werden, daß ausschließlich I entsteht.

Als Nebenreaktion wird die Umsetzung

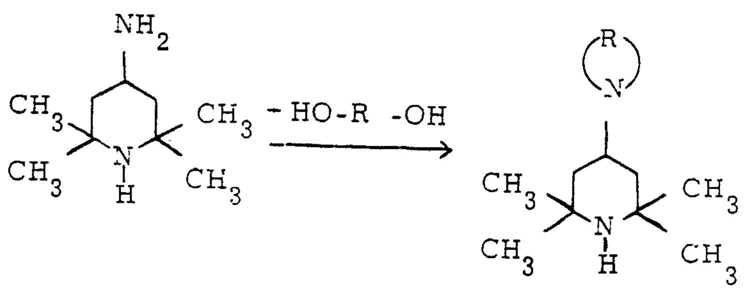


,

deren Ausmaß sowohl von der Dialkohol-Komponente als auch von den Reaktionsbedingungen abhängig ist, beobachtet. Nur in sehr untergeordnetem Maße wird die Bildung des Bis-(2,2,6,6-tetramethyl-4-piperidyl)-amins

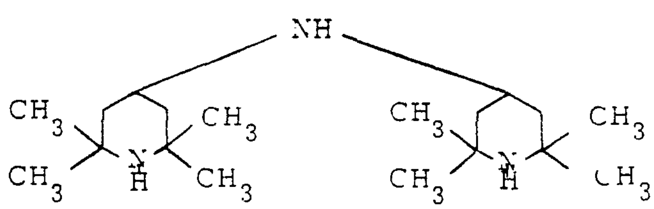

beobachtet.

Das 4-Amino-2,2,6,6-tetramethylpiperidin (II) wird vorzugsweise im Überschuß, bezogen auf die Diol-Komponente, eingesetzt.

Die Reaktionstemperaturen betragen 120 bis 250 °C, insbesondere 160 bis 220 °C, vorzugsweise 180 bis 200 °C. Geeignete Katalysatoren sind Kupferchromit-Kontakte, die vorzugsweise durch Bariumoxid-Zusatz aktiviert sind. Die Kontaktbelastung, bei bevorzugt kontinuierlicher Arbeitsweise im Rieselverfahren, beträgt 0,1 bis 3 VHSV, bezogen auf das Einsatzgemisch. Besonders vorteilhaft sind VHSV-Werte von 0,2 bis 1, insbesondere 0,2 bis 0,5.

Der erforderliche Wasserstoffdruck liegt zwischen 1 und 300 bar, bevorzugt wird der Druckbereich von 3 bis 30 bar.

Geeignete Dialkohol-Komponenten sind z. B. Ethylenglykol, 1,3-Propandiol, 1,4-Butandiol, 1,6-Hexandiol, 1,12-Dodecandiol, 2,2,4-Trimethyl-1,6-hexandiol und 2,4,4-Trimethyl-1,6-hexandiol. Bevorzugt ist 1,6-Hexandiol.

## Beispiel

N,N'-Bis-(2,2,6,6-tetramethyl-4-piperidyl)-hexamethylendiamin

In einem 1 l-Schachtofen, der mit 500 ml reduziertem Kupferchromit-Kontakt (Harshaw Cu 1107 der Fa. Harshaw, DE-5 632 Wermelskirchen) gefüllt ist, werden bei 180 °C und 30 bar Wasserstoff stündlich 250 ml eines Gemisches aus 4-Amino-2,2,6,6-tetramethylpiperidin und 1,6-Hexandiol im Molverhältnis 4 : 1 umgesetzt.

Nach GC-Analyse enthält das Austrittsprodukt nach einer Versuchsdauer von 10 Tagen

73,1 % 4-Amino-2,2,6,6-tetramethylpiperidin
9,8 % 1,6-Hexandiol
1,0 % 4-Hexamethylenimino-2,2,6,6-tetramethylpiperidin
0,55 % Bis-(2,2,6,6-tetramethyl-4-piperidyl)-amin
6,5 % 4-(6-Hydroxyhexylamino)-piperidin
9,0 % N,N'-Bis-(2,2,6,6-tetramethyl-4-piperidyl)-hexamethylendiamin.

Dies entspricht einem Umsatz an 4-Amino-2,2,6,6-tetramethylpiperidin von 14,4 % und einem 1,6-Hexandiol-Umsatz von 38,7 % sowie, unter Berücksichtigung der Rückführbarkeit von 4-Amino-2,2,6,6-tetramethylpiperidin, 1,6-Hexandiol und 4-(6-Hydroxylhexylamino)-2,2,6,6-tetramethylpiperidin, einer Ausbeute an N,N,'-Bis-(2,2,6,6-tetramethyl-4-piperidyl)-hexamethylendiamin von 89,9 % bezogen auf 4-Amino-2,2,6,6-tetramethylpiperidin, und von 92,0 %, bezogen auf 1,6-Hexandiol.

Durch destillative Aufarbeitung wurden isoliert :

4-Hexamethylenimino-2,2,6,6-tetramethylpiperidin
$Kp_{13}$ = 150 °C, $n_D^{25}$ = 1,487 0
Bis-(2,2,6,6-tetramethyl-4-piperidyl)-amin
$Kp_{19}$ = 180 °C, Fp. = 74-77 °C
4-(6-Hydroxyhexylamino)-2,2,6,6-tetramethylpiperidin
$Kp_{36}$ = 195 °C, Fp = 80-81 °C
N,N'-Bis-(2,2,6,6-tetramethyl-4-piperidyl)-hexamethylendiamin
$Kp_{37}$ = 253 °C, Fp. = 60,5-61,5 °C

Die Druckwerte der Siedepunkte wurden in mbar angegeben.

Nach 60 tätiger Versuchdauer wurde kein Aktivitätsabfall des Katalysators beobachtet.

**Patentansprüche**

1. Verfahren zur Herstellung von Polyalkylpiperidylaminen der allgemeinen Formel I

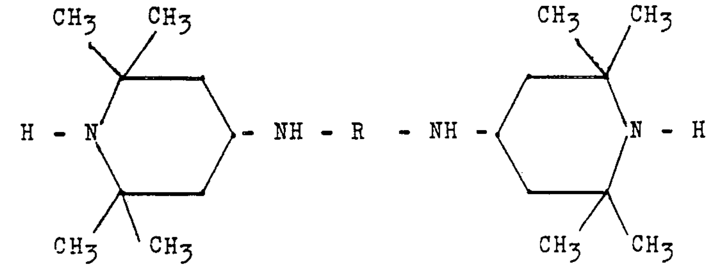

(I)

in der R einen $C_{2-18}$-Alkylenrest, der $C_{1-3}$-alkylsubstituiert sein kann, bedeutet, dadurch gekennzeichnet, daß man 4-Amino-2,2,6,6-tetramethylpiperidin der Formel II

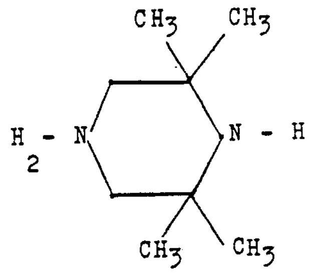

(II)

mit einem difunktionellen Alkohol der allgemeinen Formel

HO—R—OH

in der R die obige Bedeutung aufweist, bei einem Wasserstoffdruck von 1 bis 300 bar und einer Temperatur von 120 bis 250 °C in Gegenwart eines Kupferchromitkatalysators reduktiv alkyliert.

2. Verfahren nach Anspruch 1, dadurch gekennzeichnet, daß man die Umsetzung bei einem Wasserstoffdruck von 3 bis 30 bar durchführt.

3. Verfahren nach den Ansprüchen 1 und 2, dadurch gekennzeichnet, daß man 4-Amino-2,2,6,6-tetramethylpiperidin mit dem Dialkohol im Molverhältnis von 10 : 1.

4. Verfahren nach Anspruch 3, dadurch gekennzeichnet, daß man 4-Amino-2,2,6,6-tetramethylpiperidin mit dem Dialkohol im Molverhältnis von 4 : 1 bis 2 : 1 umsetzt.

5. Verfahren nach den Ansprüchen 1 bis 4, dadurch gekennzeichnet, daß man die Umsetzung bei einer Temperatur von 180 bis 200 °C durchführt.

6. Verfahren nach den Ansprüchen 1 bis 5, dadurch gekennzeichnet, daß die VHSV des Einsatzgemisches aus 4-Amino-2,2,6,6-tetramethylpiperidin und difunktionellem Alkohol 0,1 bis 3 beträgt.

7. Verfahren nach Anspruch 6, dadurch gekennzeichnet, daß die VHSV 0,2 bis 0,5 beträgt.

8. Verfahren nach den Ansprüchen 1 bis 7, dadurch gekennzeichnet, daß man als difunktionellen Alkohol 1,6-Hexandiol verwendet.

## Claims

1. A process for the production of a polyalkylpiperidylamine of the general formula

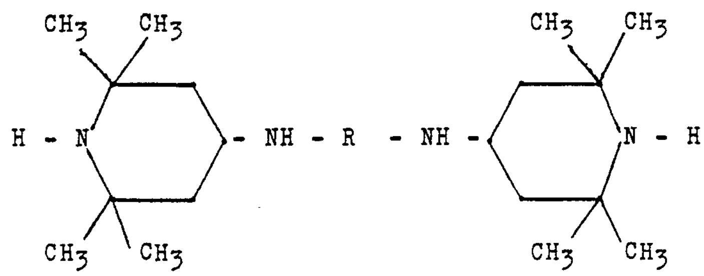

(I)

in which R is $C_2$-$C_{18}$ alkylene optionally substituted by $C_1$-$C_3$ alkyl, characterised in that 4-amino-2,2,6,6-tetramethylpiperidine of the formula II

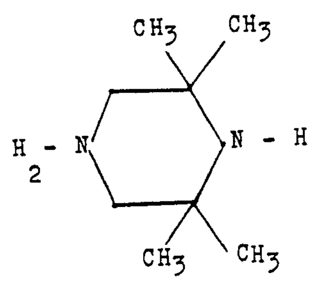

(II)

is reductively alkylated with a difunctional alcohol of the general formula

HO—R—OH

in which R has the above meaning, at a hydrogen pressure of 1 to 300 bar and a temperature of 120 to 250 °C in the presence of a copper chromite catalyst.

2. A process according to claim 1, characterised in that the reaction is carried out at a hydrogen pressure of 3 to 30 bar.

3. A process according to claim 1 or 2, characterised in that 4-amino-2,2,6,6-tetramethylpiperidine is

reacted with the dialcohol at a mole ratio of 10 : 1.

4. A process according to claim 3, charaterised in that 4-amino-2,2,6,6-tetramethylpiperidine is reacted with the dialcohol in a mole ratio of 4 : 1 to 2 : 1.

5. A process according to any of claims 1 to 4, characterised in that the reaction is carried out at a temperature of 180 to 200 °C.

6. A process according to any of claims 1 to 5, characterised in that the VHSV of the starting mixture of 4-amino-2,2,6,6-tetramethylpiperidine and difunctional alcohol is 0.1 to 3.

7. A process according to claim 6, characterised in that the VHSV is 0.2 to 0.5.

8. A process according to any of claims 1 to 7, characterised in that 1,6-hexanediol is used as difunctional alcohol.

**Revendications**

1. Procédé de préparation de poly-alkyl-pipéridylamines de la formule générale (I)

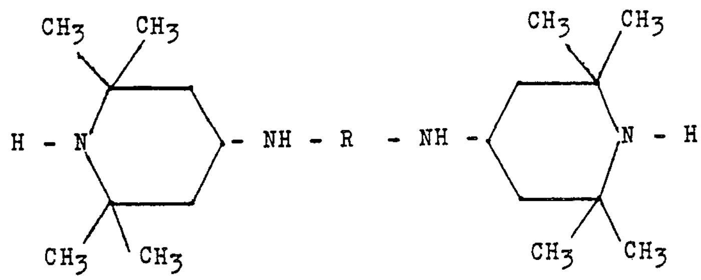

(I)

dans laquelle R représente un radical alkylène comportant de 2 à 18 atomes de carbone et pouvant être substitué par un reste alkyle comportant de 1 à 3 atomes de carbone, caractérisé par le fait que sous une pression d'hydrogène de 1 à 300 bars et à une température de 120 à 250 °C, on alkyle par réduction, en présence d'un catalyseur au chromite de cuivre, une 4-amino-2,2,6,6-tétra-méthyl-pipéridine de la formule (II)

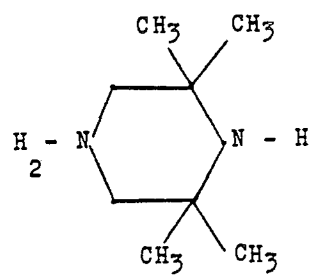

(II)

avec un dialcool de la formule générale

HO—R—OH

dans laquelle R présente la signification donnée ci-dessus.

2. Procédé selon la revendication 1, caractérisé par le fait qu'on effectue la réaction sous une pression d'hydrogène de 3 à 30 bars.

3. Procédé selon les revendications 1 et 2, caractérisé par le fait que l'on fait réagir la 4-amino-2,2,6,6-tétraméthyl-pipéridine sur le dialcool dans un rapport molaire de 10 : 1.

4. Procédé selon la revendication 3, caractérisé par le fait que l'on fait réagir la 4-amino-2,2,6,6-tétraméthyl-pipéridine sur le dialcool dans un rapport molaire de 4 : 1 à 2 : 1.

5. Procédé selon les revendications 1 à 4, caractérisé par le fait que l'on effectue la réaction à une température de 180 à 200 °C.

6. Procédé selon les revendications 1 à 5, caractérisé par le fait que la valeur VHSV du mélange utilisé de 4-amino-2,2,6,6-tétraméthyl-pipéridine et de dialcool est de 0,1 à 3.

7. Procédé selon la revendication 6, caractérisé par le fait que la valeur VSHV est de 0,2 à 0,5.

8. Procédé selon les revendications 1 à 7, caractérisé par le fait que l'on utilise, comme dialcool, le 1,6-hexane-diol.